(19) Europäisches Patentamt
European Patent Office
Office européen des brevets

(11) Veröffentlichungsnummer: **0 000 928**
**B1**

(12) **EUROPÄISCHE PATENTSCHRIFT**

(45) Veröffentlichungstag der Patentschrift: **18.03.81**

(21) Anmeldenummer: **78100690.3**

(22) Anmeldetag: **17.08.78**

(51) Int. Cl.³: **C 07 D 233/91,** C 07 D 403/06, C 07 D 401/06, C 07 D 413/06, C 07 D 401/12, A 61 K 31/415

(54) **Neue Nitroimidazole und diese enthaltende pharmazeutische Präparate sowie deren Herstellung.**

(30) Priorität: **19.08.77 GB 3490877**
**15.05.78 GB 1953478**

(43) Veröffentlichungstag der Anmeldung:
**07.03.79 Patentblatt 79/5**

(45) Bekanntmachung des Hinweises auf die Patenterteilung:
**18.03.81 Patentblatt 81/11**

(84) Benannte Vertragsstaaten:
**BE CH DE FR GB LU NL SE**

(56) Entgegenhaltungen:
**CH-A-513 879**
**DE-A-1 695 131**
**DE-A-2 410 749**

(73) Patentinhaber: **F. HOFFMANN-LA ROCHE & CO. Aktiengesellschaft, CH-4002 Basel (CH)**

(72) Erfinder: **Smithen, Carey Ernest, 17 The Valley Green, Welwyn Garden City Herts (GB)**

(74) Vertreter: ***Lederer, Franz, Dr. et al,* Patentanwälte Lederer, Meyer Lucile-Grahn-Strasse 22, D-8000 München 80 (DE)**

EP 0 000 928 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

BUNDESDRUCKEREI BERLIN

## Neue Nitroimidazole und diese enthaltende pharmazeutische Präparate sowie deren Herstellung

Die vorliegende Erfindung betrifft neue Nitroimidazole, ein Verfahren zu deren Herstellung und pharmazeutische Präparate mit einem Gehalt an diesen Verbindungen.

Die Nitroimidazole der vorliegenden Erfindung sind Verbindungen der allgemeinen Formel

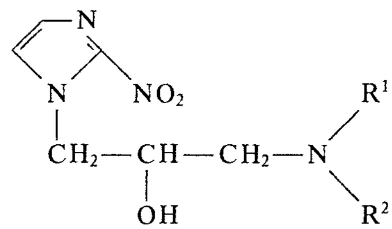

(I)

worin

$R^1$ Wasserstoff, Niederalkyl, Hydroxy-niederalkyl, Niedercycloalkyl, Aryl oder Aryl-niederalkyl

$R^2$ Niederalkyl, Hydroxy-niederalkyl, Niedercycloalkyl, Aryl, Aryl-niederalkyl oder ein Rest der Formel

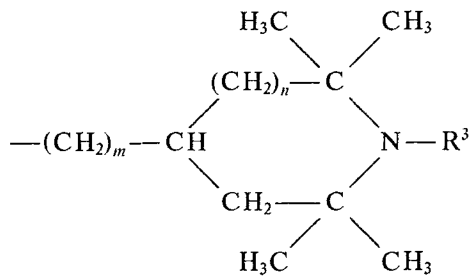

mit m = 0 und n = 1 oder
m = 1 und n = 0 und worin

$R^3$ Wasserstoff, Methyl, Hydroxy oder ein freies Sauerstoffradikal oder

$R^1$ und $R^2$ zusammen mit dem Stickstoffatom, an das sie gebunden sind, einen 5-, 6- oder 7gliedrigen gesättigten heteromonocyclischen Ring, der eine Hydroxygruppe an einem dem Stickstoffatom nicht direkt benachbarten C-Atom tragen kann und der ein weiteres Sauerstoff-, Schwefel- oder Stickstoffatom enthalten kann, das ggf. durch eine Niederalkyl-, Hydroxy-niederalkyl-, Aryl- oder Aryl-niederalkyl-gruppe substituiert ist, darstellen,

und deren Säureadditionssalze.

In der Beschreibung und den Ansprüchen bedeutet der Ausdruck »Niederalkyl« eine gerad- oder verzweigtkettige Alkylgruppe mit vorzugsweise 1—6 Kohlenstoffatomen, wie Methyl, Äthyl, Propyl, Isopropyl, Butyl, tert.Butyl, Pentyl und Hexyl. Beispiele von Hydroxyniederalkylgruppen sind Hydroxymethyl, 2-Hydroxyäthyl, 2-Hydroxypropyl, 3-Hydroxypropyl und 2-Hydroxybutyl.

Der Ausdruck »Niedercycloalkyl« bedeutet Cycloalkylgruppen mit vorzugsweise bis zu 6 Kohlenstoffatomen, wie Cyclopropyl, Cyclopentyl und Cyclohexyl. Der Ausdruck »Aryl« bedeutet die Phenylgruppe oder die ein- oder mehrfach, vorzugsweise ein- oder zweifach substituierte Phenylgruppe, wobei die Substituenten Halogene (d. h. Fluor, Chlor, Brom oder Jod) Trifluormethyl, Niederalkyl, Niederalkoxy, Nitro und Amino sein können. Beispiele solcher substituierter Phenylgruppen sind 4-Chlorphenyl, 2,4-Dichlorphenyl, p-Tolyl, 4-Methoxyphenyl, 4-Nitrophenyl und 4-Aminophenyl. Der Ausdruck »Aryl-niederalkyl« bezeichnet einen durch eine Arylgruppe substituierten Niederalkylrest. Beispiele solcher Arylniederalkylgruppen sind Benzyl, 4-Chlorbenzyl, Phenäthyl und Phenylpropyl. Beispiele von gesättigten heteromonocyclischen Ringen, die aus den Substituenten $R^1$ und $R^2$ zusammen mit dem Stickstoffatom gebildet werden und die eine Hydroxygruppe an einem dem Stickstoffatom nicht direkt benachbarten C-Atom tragen können, sind Pyrrolidino, Piperidino, 3-Hydroxy-pyrrolidino, 4-Hydroxy-piperidino und 3-Hydroxy-hexahydro-1H-azepino. Beispiele von gesättigten heteromonocyclischen Ringen, die aus den Substituenten $R^1$ und $R^2$ zusammen mit dem Stickstoffatom, an das sie gebunden sind, gebildet werden und die ein weiteres Sauerstoff-, Schwefel- oder Stickstoffatom enthalten können, das gegebenenfalls wie vorstehend angegeben substituiert sein kann, sind Piperazino, N-Methylpiperazino, N-(2-Hydroxyäthyl)-piperazino, Morpholino und Thiamorpholino. Der Ausdruck »Niederalkoxy« bedeutet eine gerad- oder verzweigtkettige Alkoxygruppe mit vorzugsweise 1—6 Kohlenstoffatomen, wie Methoxy oder Äthoxy.

Eine interessante Gruppe von Nitroimidazolen der vorliegenden Erfindung sind Verbindungen der Formel I in denen $R^1$ Wasserstoff, Niederalkyl, Hydroxy-niederalkyl, Aryl oder Aryl-niederalkyl, $R^2$ Niederalkyl, Hydroxy-niederalkyl, Aryl oder Aryl-niederalkyl oder $R^1$ und $R^2$ zusammen mit dem Stickstoffatom, an das sie gebunden sind, einen 5-, 6- oder 7-gliedrigen gesättigten heteromonocyclischen Ring darstellen, der ein weiteres Sauerstoff-, Schwefel- oder Stickstoffatom enthalten kann, das gegebenenfalls durch eine Niederalkyl-, Hydroxy-niederalkyl-, Aryl oder Aryl-niederalkyl-gruppe substituiert sein kann, sowie deren Säureadditionssalze.

Eine besonders interessante Gruppe von Nitroimidazolderivaten der vorliegenden Erfindung sind solche Verbindungen der Formel I in denen $R^1$ und $R^2$ zusammen mit dem Stickstoffatom, an das sie gebunden sind, einen 6-gliedrigen heteromonocyclischen Ring darstellen, der ein weiteres Sauerstoff- oder Stickstoffatom enthalten kann, das gegebenenfalls durch eine Niederalkylgruppe substituiert ist, sowie deren Säureadditionssalze.

Beispiele von erfindungsgemäßen Verbindungen der Formel I sind:

2-Nitro-α-(piperidino)-methyl-1-imidazol-äthanol,
α-(Morpholino)-methyl-2-nitro-1-imidazol-äthanol,
α-(4-Methylpiperazino)-methyl-2-nitro-1-imidazol-äthanol,
2-Nitro-α-(pyrrolidino)-methyl-1-imidazol-äthanol,
α-(Diäthylamino)-methyl-2-nitro-1-imidazol-äthanol,
α-[Di-(2-hydroxyäthyl)-amino]-methyl-2-nitro-1-imidazol-äthanol,
α-(tert.Butylamino)-methyl-2-nitro-1-imidazol-äthanol,
α-(Benzylamino)-methyl-2-nitro-1-imidazol-äthanol,
α-[(4-Methoxyphenyl)-amino]-methyl-2-nitro-1-imidazol-äthanol,
α-(Dimethylamino)-methyl-2-nitro-1-imidazol-äthanol,
α-(Hexahydro-1H-azepino)-methyl-2-nitro-1-imidazol-äthanol,
4-[2-Hydroxy-3-(2-nitro-1-imidazolyl)-propylamino]-2,2,6,6-tetramethylpiperidin-N-oxyl,
α-[(2,2,6,6-Tetramethyl-4-piperidinyl)-amino]-methyl-2-nitro-1-imidazol-äthanol,
α-(Cyclohexylamino)-methyl-2-nitro-1-imidazol-äthanol,
α-(Dicyclohexylamino)-methyl-2-nitro-1-imidazol-äthanol und
1-[2-Hydroxy-3-(2-nitro-1-imidazolyl)-propyl]-3-pyrrolidinol.

Die erfindungsgemäßen Nitroimidazole und ihre Säureadditionssalze können dadurch hergestellt werden, daß man

(a) ein Epoxid der Formel

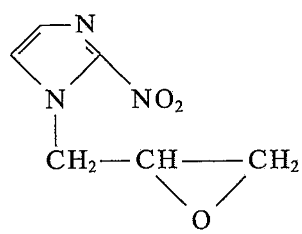

(II)

mit einem Amin der Formel

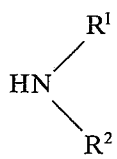

(III)

worin $R^1$ und $R^2$ wie vorstehend definiert sind, umsetzt oder

(b) eine Verbindung der Formel

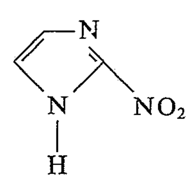

(IV)

mit einem Epoxid der Formel

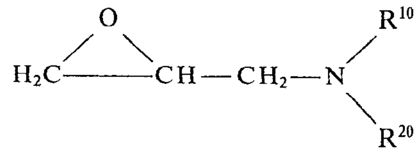

(V)

worin $R^{10}$ und $R^{20}$ ebenso definiert sind wie $R^1$ und $R^2$ mit dem Unterschied, daß $R^{10}$ nicht Wasserstoff ist, in Gegenwart einer Base umsetzt oder

(c) ein Halohydrin der Formel

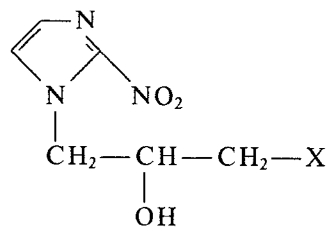

(VI)

worin X Chlor oder Brom darstellt,
mit einem Amin der Formel III umsetzt und gewünschtenfalls eine erhaltene Verbindung der Formel I in ein Säureadditionssalz überführt.

Die Umsetzung eines Epoxids der Formel II mit einem Amin der Formel III gemäß Verfahrensvariante (a) kann in Gegenwart oder Abwesenheit eines inerten organischen Lösungsmittels durchgeführt werden. Geeignete inerte organische Lösungsmittel sind niedere Alkanole (z. B. Methanol und Äthanol), Dimethylformamid oder Dimethylacetamid. Andererseits kann ein Überschuß eines Amins der Formel III als Lösungsmittel verwendet werden. Druck und Temperatur während der Reaktion sind nicht kritisch; die Umsetzung kann bei Raumtemperatur und unter Atmosphärendruck oder aber bei erhöhter Temperatur und/oder erhöhtem Druck durchgeführt werden. In einer bevorzugten Ausführungsform wird die Reaktion bei einer Temperatur von etwa 50° C bis zur Rückflußtemperatur des Reaktionsgemisches und unter Normaldruck durchgeführt.

Die Kondensation einer Verbindung der Formel IV (Azomycin) mit einem Epoxid der Formel V gemäß Verfahrensvariante (b) wird in Gegenwart einer Base durchgeführt. Es werden vorzugsweise katalytische Mengen der Base verwendet, obgleich auch größere Mengen verwendet werden können. Bevorzugte Basen sind Alkalimetallcarbonate (z. B. Natriumcarbonat oder Kaliumcarbonat), obgleich auch andere Basen, wie Alkalimetallhydroxide (z. B. Natriumhydroxid oder Kaliumhydroxid), verwendet werden können. Die Kondensation wird zweckmäßigerweise in Gegenwart eines inerten organischen Lösungsmittels, z. B. eines niederen Alkanols (z. B. Methanol oder Äthanol), durchgeführt. Obgleich die Kondensation auch bei Raumtemperatur und unter höherem Druck durchgeführt werden kann, wird sie vorzugsweise bei erhöhter Temperatur, insbesondere bei der Rückflußtemperatur des Reaktionsgemisches, und unter Atmosphärendruck durchgeführt.

Bei der Umsetzung eines Halohydrins der Formel VI mit einem Amin der Formel III gemäß Verfahrensvariante (c) wird zweckmäßigerweise mindestens ein Mol Amin pro Mol Halohydrin eingesetzt. Die Reaktion wird zweckmäßigerweise in Gegenwart eines säurebindenden Mittels, wie eines Alkalimetallcarbonats (z. B. Natriumcarbonat oder Kaliumcarbonat) oder eines tertiären organischen Amins (z. B. Pyridin) oder, vorzugsweise, in Gegenwart von überschüssigem Amin der Formel III durchgeführt. Demgemäß werden in einer bevorzugten Ausführungsform mindestens 2 Mol eines Amins der Formel III pro Mol des Halohydrins der Formel VI eingesetzt. Die Umsetzung findet zweckmäßigerweise in Gegenwart eines inerten organischen Lösungsmittels, beispielsweise eines niederen Alkanols (z. B. Methanol oder Äthanol), statt. Die Temperatur und der Druck während der Reaktion sind nicht kritisch. Es kann bei Raumtemperatur und unter Normaldruck oder aber bei erhöhter Temperatur und unter Druck gearbeitet werden. In einer bevorzugten Ausführungsform wird die Reaktion bei erhöhter Temperatur, insbesondere bei Rückflußtemperatur des Reaktionsgemisches, und unter Normaldruck ausgeführt. Als bevorzugtes Halohydrin der Formel VI wird Chlorhydrin verwendet.

Die Verbindungen der Formel I können in Säureadditionssalze übergeführt werden, z. B. durch Umsetzung mit einer anorganischen Säure, wie einer Halogenwasserstoffsäure (z. B. HCl oder HBr), Schwefelsäure, Salpetersäure oder Phosphorsäure oder mit einer organischen Säure, wie Essigsäure, Zitronensäure, Maleinsäure, Äpfelsäure, Fumarsäure, Bernsteinsäure, Methansulfonsäure oder Paratoluolsulfonsäure. Physiologisch verträgliche Säureadditionssalze, insbesondere die Hydrochloride, sind bevorzugt. Physiologisch nicht verträgliche Säureadditionssalze können in physiologisch verträgliche Säureadditionssalze durch Behandlung mit einer Base und anschließende Umsetzung mit

einer physiologisch verträglichen Säure übergeführt werden.

Die Ausgangsverbindungen der Formeln II bis VI sind bekannte Verbindungen.

Die Verbindungen der Formel I und ihre physiologisch verträglichen Säureadditionssalze können als sogenannte »Radiosensitiser« zur Sensibilisierung hypoxischer Zellen gegenüber Strahlen verwendet werden, insbesondere im Zusammenhang mit der Behandlung von hypoxischen Tumorzellen durch Bestrahlung. Die Wirksamkeit der Verbindungen der Formel I und ihrer physiologisch verträglichen Säureadditionssalze als Radiosensitiser für hypoxische Zellen kann in einem in vitro-Versuch an hypoxischen chinesischen Hamster-V 79-Zellen (vergl. Adams et al., Radiation Research, 67, 9—20 (1976) gezeigt werden. So besitzen z. B. 2-Nitro-α-(piperidino)-methyl-1-imidazol-äthanol-hydrochlorid und α-(Benzylamino)-methyl-2-nitro-1-imidazol-äthanol-hydrochlorid, zwei neue erfindungsgemäße Nitroimidazole, einen Verstärkungsfaktor von 1,6 (VF Index 1,6) bei Konzentrationen von 30 μMol bzw. 40 μMol. Um die gleiche Verstärkung mit Misonidazol oder Metronidazol zu erreichen, müßten diese beiden bekannten Nitroimidazole in einer Konzentration von 300 μMol bzw. 4000 μMol verwendet werden.

Die Verbindungen der Formel I und ihre physiologisch verträglichen Säureadditionssalze können auch zur Bekämpfung von durch Protozoen verursachten Infektionen verwendet werden, insbesondere von Infektionen, die durch Trichomonas vaginalis verursacht werden.

Die erfindungsgemäßen Verbindungen können als pharmazeutische Präparate mit direkter oder verzögerter Freigabe des Wirkstoffs in Mischung mit einem für die enterale, z. B. orale, oder parenterale Applikation geeigneten organischen oder anorganischen inerten Trägermaterial, wie z. B. Wasser, Gelatine, Gummi arabicum, Milchzucker, Stärke, Magnesiumstearat, Talk, pflanzlichen Ölen, Polyalkylenglycolen oder Vaseline verwendet werden. Die Präparate können in fester Form, z. B. als Tabletten, Dragées, Suppositorien, Kapseln; in halbfester Form, z. B. als Salben; oder in flüssiger Form, z. B. als Lösungen, Suspensionen oder Emulsionen vorliegen. Gegebenenfalls sind sie sterilisiert und bzw. oder enthalten weitere Hilfsstoffe, wie Konservierungs-, Stabilisierungs-, Netz- oder Emulgiermittel, Mittel zur geschmacklichen Verbesserung, Salze zur Veränderung des osmotischen Drucks oder Puffersubstanzen. Die Präparate können auch andere therapeutisch wertvolle Verbindungen enthalten.

Die Herstellung der pharmazeutischen Präparate kann in der jedem Fachmann geläufigen Weise erfolgen, indem man eine der erfindungsgemäßen Verbindungen als aktiven Bestandteil mit einem zur therapeutischen Verabreichung geeigneten, inerten, festen oder flüssigen Träger vermischt, und das Gemisch gegebenenfalls in eine besondere galenische Form bringt.

Bei Verwendung als Radiosensitiser können die erfindungsgemäßen Verbindungen oral bei einer Dosierung von etwa 20 bis 60 mg pro kg Körpergewicht und Tag verabreicht werden. Im allgemeinen sollte aber die während einer Behandlung verabreichte Gesamtdosis etwa 200 mg pro kg Körpergewicht und Tag nicht überschreiten. Bei Verwendung als Mittel gegen Protozoen, können die erfindungsgemäßen Verbindungen ebenfalls bei einer täglichen Dosierung von etwa 20 bis 60 mg pro kg Körpergewicht oral verabreicht werden.

Es versteht sich jedoch von selbst, daß die vorstehend genannten Dosisbereiche nur Richtlinien darstellen und je nach den individuellen Erfordernissen über- sowie unterschritten werden können.

### Beispiel 1

(a) Ein Gemisch aus 5,1 g 1-(2,3-Epoxypropyl)-2-nitroimidazol, 3,3 g Diäthylamin und 100 ml Methanol wurde 12 bis 18 Stunden unter Rückfluß erhitzt. Nach Abzug des Lösungsmittels unter vermindertem Druck wurden 8,1 g eines hellbraunen Rückstandes erhalten, der in 25 ml heißem Äthanol gelöst wurde. Die Lösung wurde mit Holzkohle entfärbt und filtriert. Nach Kristallisation wurden 5,2 g (72%) α-(Diäthylamino)-methyl-2-nitro-1-imidazoläthanol in Form hellgelber Kristalle, F. 92—93° C, erhalten.

(b) 3,6 g α-(Diäthylamino)-methyl-2-nitro-1-imidazol-äthanol wurden in einer minimalen Menge warmen Äthanols gelöst und mit einem geringen Überschuß von wasserfreiem ätherischem HCl behandelt. Das nach Abkühlung und Kristallisation erhaltene cremefarbene Hydrochloridsalz (4,0 g) wurde in ca. 40 ml heißem Äthanol gelöst, zur Entfärbung mit Holzkohle behandelt, filtriert und, falls nötig, mit einigen ml trockenem Diäthyläther zur Kristallisation gebracht. Es wurden so 4,0 g α-(Diäthylamino)-methyl-2-nitro-1-imidazol-äthanol-hydrochlorid in Form schwach cremefarbener Kristalle, F. 145—146° C (Zersetzung), erhalten.

### Beispiel 2

(a) In zu Beispiel 1 (a) analoger Weise wurde nach Kristallisation aus Isopropanol 2-Nitro-α-(pyrrolidino)-methyl-1-imidazol-äthanol in Form hellgelber Kristalle, F. 83—85° C, erhalten. Ausbeute 79%.

(b) In zu Beispiel 1 (b) analoger Weise wurde 2-Nitro-α-(pyrrolidino)-methyl-1-imidazol-äthanol-hydrochlorid in Form schwach cremefarbener Kristalle, F. 158—159° C (Zers.), erhalten. Ausbeute 87%.

### Beispiel 3

(a) In zu Beispiel 1 (a) analoger Weise wurde nach Kristallisation aus Äthanol 2-Nitro-α-(piperidino)-methyl-1-imidazol-äthanol in Form hellgelber Kristalle, F. 110—112° C, erhalten. Ausbeute 88%.

(b) In zu Beispiel 1 (b) analoger Weise wurde 2-Nitro-α-(piperidino)-methyl-1-imidazol-äthanol-hydrochlorid in Form schwach cremefarbener Kristalle, F. 144—145° C (Zers.), erhalten. Ausbeute 90%.

Beispiel 4

(a) In zu Beispiel 1 (a) analoger Weise wurde nach Kristallisation aus Äthanol, α-(Morpholino)-methyl-2-nitro-1-imidazol-äthanol in Form hellgelber Kristalle, F. 112—113° C, erhalten. Ausbeute 88%.

(b) In zu Beispiel 1 (b) analoger Weise wurde α-(Morpholino)-methyl-2-nitro-1-imidazol-äthanol-hydrochlorid in Form schwach cremefarbener Kristalle, F. 196—197° C (Zers.), erhalten. Ausbeute 93%.

Beispiel 5

(a) In zu Beispiel 1 (a) analoger Weise wurde nach Kristallisation aus Äthanol, α-(4-Methylpiperazino)-methyl-2-nitro-1-imidazol-äthanol in Form schwach gelber Kristalle, F. 144—145° C, erhalten. Ausbeute 62%.

(b) In zu Beispiel 1 (b) analoger Weise wurde α-(4-Methyl-piperazino)-methyl-2-nitro-1-imidazol-äthanol-dihydrochlorid in Form fast farbloser Kristalle, F. 215—216° C, (Zers.), erhalten. Ausbeute 93%.

Beispiel 6

(a) In zu Beispiel 1 (a) analoger Weise, aber unter Verwendung äquimolekularer Mengen der Reagentien, wurde, nach Kristallisation aus Isopropanol, α-[Di-(2-hydroxyäthyl)-amino]-methyl-2-nitro-1-imidazol-äthanol in Form hellgelber Kristalle, F. 92—93° C, erhalten. Ausbeute 79%.

(b) In zu Beispiel 1 (b) analoger Weise wurde α-[Di-(2-hydroxyäthyl)-amino]-methyl-2-nitro-1-imidazol-äthanol-hydrochlorid in Form schwach cremefarbener Kristalle, F. 151—152° C (Zers.), erhalten. Ausbeute 75%.

Beispiel 7

(a) In zu Beispiel 1 (a) analoger Weise, aber unter Verwendung von 2 Moläquivalenten von tert.-Butylamin, wurde, nach Kristallisation aus Äthanol, α-(tert.-Butylamino)-methyl-2-nitro-1-imidazol-äthanol in Form hellgelber Kristalle, F. 114—115° C, erhalten. Ausbeute 36%.

(b) In zu Beispiel 1 (b) analoger Weise wurde α-(tert.-Butylamino)-methyl-2-nitro-1-imidazol-äthanol-hydrochlorid in Form schwach cremefarbener Kristalle, F. 198—199° C (Zers.), erhalten. Ausbeute 87%.

Beispiel 8

(a) In zu Beispiel 1 (a) analoger Weise, aber unter Verwendung äquimolekularer Mengen der Reagentien wurde α-(Benzylamino)-methyl-2-nitro-1-imidazol-äthanol in Form eines hellgelben Gummis, der gemäß Dünnschichtchromatographie einheitlich war, erhalten.

(b) 4,5 g α-(Benzylamino)-methyl-2-nitro-1-imidazoläthanol wurden in einer minimalen Menge warmen Äthanols gelöst und mit einer äquivalenten Menge äthanolischer Maleinsäure (1,9 g) behandelt. Man ließ das Gemisch abkühlen und während mehreren Stunden auskristallisieren. Das erhaltene cremefarbene Maleinsäuresalz (5,2 g) wurde in 50 ml heißem Äthanol gelöst, mit Holzkohle entfärbt, filtriert und, soweit nötig, mit einigen ml trockenem Diäthyläther zur Kristallisation gebracht. Es wurden so 3,6 g α-(Benzylamino)-methyl-2-nitro-1-imidazol-äthanolmaleat in Form schwach cremefarbener Kristalle, F. 153—154° C (Zers.), erhalten.

(c) In analoger Weise wurde, nach Kristallisation aus Äthanol, α-(Benzylamino)-methyl-2-nitro-1-imidazol-äthanoloxalat in Form farbloser Kristalle, F. 197—198° C (Zers.), erhalten.

Beispiel 9

(a) In zu Beispiel 1 (a) analoger Weise, aber unter Verwendung äquimolarer Mengen der Reagentien wurde, nach Kristallisation aus Äthanol, α-[(4-Methoxyphenyl)-amino]-methyl-2-nitro-1-imidazol-äthanol in Form brauner Nadeln, F. 162—163° C, erhalten. Ausbeute 80%.

(b) In zu Beispiel 1 (b) analoger Weise wurde α-[(4-Methoxyphenyl)-amino]-methyl-2-nitro-1-imidazol-äthanolhydrochlorid in Form sehr schwach rosagefärbter Kristalle, F. 156—157° C (Zers.), erhalten. Ausbeute 97%.

Beispiel 10

(a) In zu Beispiel 1 (a) analoger Weise wurde, nach Umkristallisation aus Isopropanol, α-(Dimethylamino)-methyl-2-nitro-1-imidazol-äthanol in Form hellgelber Kristalle, F. 78—79° C, erhalten. Ausbeute 40%.

(b) In zu Beispiel 1 (b) analoger Weise wurde, nach Umkristallisation aus Methanol/Diäthyläther, α-(Dimethyl-amino)-methyl-2-nitro-1-imidazol-äthanol-hydrochlorid in Form farbloser Kristalle, F. 202—203° C (Zers.), erhalten.

### Beispiel 11

(a) In zu Beispiel 1 (a) analoger Weise wurde, nach Umkristallisation aus Isopropanol, α-(Hexahydro-1 H-azepino)-methyl-2-nitro-1-imidazol-äthanol in Form hellgelber Kristalle, F. 102—103° C, erhalten.

(b) In zu Beispiel 1 (b) analoger Weise wurde, nach Umkristallisation aus Äthanol/Diäthyläther, α-(Hexahydro-1 H-azepino)-methyl-2-nitro-1-imidazol-äthanol-hydrochlorid in Form farbloser Kristalle, F. 133—134° C (Zers.), erhalten.

### Beispiel 12

In zu Beispiel 1 (a) analoger Weise wurde in 62%iger Ausbeute, nach Chromatographie an Aluminiumoxid (Elution mit Dichlormethan), 4-[2-Hydroxy-3-(2-nitro-1-imidazolyl)-propylamino]-2,2,6,6-tetramethylpiperidin-N-oxyl in Form orangefarbener Kristalle, F. 150—151° C, erhalten.

### Beispiel 13

In zu Beispiel 1 (a) analoger Weise wurde, nach Umkristallisation aus Isopropanol, α-[(2,2,6,6-Tetramethyl-4-piperidinyl)-amino]-methyl-2-nitro-1-imidazol-äthanol in Form cremefarbener Kristalle, F. 151—153° C, erhalten. Ausbeute 60%.

### Beispiel 14

(a) In zu Beispiel 1 (a) analoger Weise wurde, nach Umkristallisation aus Isopropanol, α-(Cyclohexylamino)-methyl-2-nitro-1-imidazol-äthanol in Form cremefarbener Kristalle, F. 66—68° C, erhalten. Ausbeute 90%.

(b) In zu Beispiel 1 (b) analoger Weise wurde, nach Umkristallisation aus Äthanol/Diäthyläther, α-(Cyclohexylamino)-methyl-2-nitro-1-imidazol-äthanol-hydrochlorid in Form farbloser Kristalle, F. 192—193° C (Zers.), erhalten.

### Beispiel 15

(a) In zu Beispiel 1 (a) analoger Weise wurde, nach Umkristallisation aus Äthanol, α-(Dicyclohexylamino)-methyl-2-nitro-1-imidazol-äthanol in Form orangegelber Kristalle, F. 149—150° C, erhalten. Ausbeute 31%.

(b) In zu Beispiel 1 (b) analoger Weise wurde, nach Umkristallisation aus Äthanol/Diäthyläther, α-(Dicyclohexylamino)-methyl-2-nitro-1-imidazol-äthanol-hydrochlorid in Form cremefarbener Kristalle, F. 208—209° C (Zers.), erhalten.

### Beispiel 16

In zu Beispiel 1 (a) analoger Weise wurde, nach Umkristallisation aus Äthanol, 1-[2-Hydroxy-3-(2-nitro-1-imidazolyl)-propyl]-3-pyrrolidinol in Form cremefarbener Kristalle, F. 126—127° C, erhalten.

### Beispiel 17

(a) Ein Gemisch aus 5,65 g 2-Nitroimidazol und 250 mg wasserfreiem Kaliumcarbonat in 150 ml Äthanol wurde 15 Minuten unter Rückfluß erhitzt. Nach Zusatz von 7,05 g frisch destilliertem 3-Piperidino-propylen-oxid in einer minimalen Menge Äthanol wurde das Gemisch weitere 3 Stunden unter Rückfluß erhitzt und anschließend filtriert. Das Filtrat wurde unter vermindertem Druck zur Trockene eingeengt und lieferte 13 g eines gelben Öles, das zwischen 100 ml Äthylacetat und 100 ml Wasser verteilt wurde. Die wäßrige Phase wurde abgetrennt, einmal mit 50 ml Äthylacetat gewaschen und die vereinigten organischen Phasen wurden dann 4mal mit jeweils 50 ml 2 N HCl extrahiert. Der vereinigte wäßrige Extrakt wurde durch Zusatz von überschüssigem festen Natriumcarbonat basisch gemacht und 3mal mit jeweils 100 ml Dichlormethan extrahiert. Der organische Extrakt wurde über wasserfreiem Natriumcarbonat getrocknet und filtriert. Das Filtrat wurde unter vermindertem Druck zur Trockene eingeengt und lieferte 6,5 g eines hellgelben Feststoffes, der in 25 ml heißem Äthanol gelöst wurde. Die Lösung wurde durch Behandlung mit Holzkohle entfärbt, filtriert und lieferte nach Kristallisation 1,4 g (11%) 2-Nitro-α-(piperidino)-methyl-1-imidazol-äthanol in Form hellgelber Kristalle, F. 108—109° C.

(b) 1,27 g 2-Nitro-α-(piperidino)-methyl-1-imidazol-äthanol wurden in 25 ml warmem Äthanol gelöst und mit einem geringen Überschuß an wasserfreiem ätherischem HCl behandelt. Man ließ das Gemisch abkühlen und während mehreren Stunden kristallisieren. Es wurden so 1,4 g 2-Nitro-α-(piperidino)-methyl-1-imidazol-äthanol-hydrochlorid in Form schwach cremefarbener Kristalle erhalten, die mit dem gemäß Beispiel 3 (b) erhaltenen Material identisch waren.

### Beispiel 18

Ein Gemisch aus 4,1 g 3-Chlor-1-(2-nitro-imidazolyl)-2-propanol, 3,4 g Piperidin und 75 ml Methanol wurde 12 bis 18 Stunden unter Rückfluß erhitzt. Unter vermindertem Druck wurde das Lösungsmittel abgezogen und der cremefarbene Rückstand (7,3 g) wurde in 75 ml Wasser unter Zusatz eines geringen Überschusses von 2 N HCl suspendiert. Die homogene Lösung wurde 3mal mit je 25 ml Dichlormethan gewaschen, mit einem geringen Überschuß einer 2 N Natriumhydroxid-Lösung

behandelt und 3mal mit jeweils 75 ml Dichlormethan extrahiert. Die vereinigten Dichlormethanextrakte wurden über wasserfreiem Magnesiumsulfat getrocknet, filtriert und unter vermindertem Druck zur Trockene eingeengt. Es wurden 4,3 g eines cremefarbenen Feststoffes erhalten, der in 25 ml heißem Äthanol gelöst wurde. Die Lösung wurde mittels Holzkohle entfärbt, filtriert und lieferte nach Kristallisation 3,3 g (65%) 2-Nitro-α-(piperidino)-methyl-1-imidazoläthanol in Form hellgelber Kristalle, F. 110—112° C. Dieses Produkt war mit dem gemäß Beispiel 3 (a) erhaltenen identisch und wurde in zu Beispiel 1 (b) analoger Weise in das Hydrochlorid übergeführt.

Beispiel A

Kapseln enthaltend:

| | per Kapsel |
|---|---|
| Aktive Substanz (Nitroimidazol der Formel I oder Säureadditionssalze davon) | 500,00 mg |
| Cellulose | 10,00 mg |
| Methylhydroxypropylcellulose | 5,00 mg |
| Dioctyl-natrium-sulfosuccinat | 1,00 mg |
| Maisstärke | 12,00 mg |
| Magnesiumstearat | 2,00 mg |
| Gesamtgewicht | 530,000 mg |

Das vorstehend beschriebene pharmazeutische Präparat kann in an sich bekannter Weise, möglichst unter Lichtausschluß, hergestellt werden und sollte im Dunkeln aufbewahrt werden.

**Patentansprüche**

1. Verfahren zur Herstellung von Nitroimidazolen der Formel

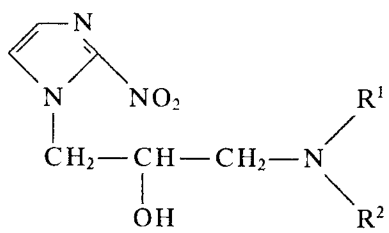

(I)

worin

$R^1$ Wasserstoff, Niederalkyl, Hydroxy-niederalkyl, Niedercycloalkyl, Aryl oder Arylniederalkyl

$R^2$ Niederalkyl, Hydroxy-niederalkyl, Niedercycloalkyl, Aryl, Aryl-niederalkyl oder ein Rest der Formel

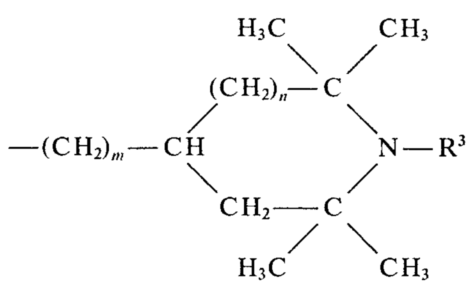

mit m = 0 und n = 1 oder
m = 1 und n = 0 und worin

$R^3$ Wasserstoff, Methyl, Hydroxy oder ein freies Sauerstoffradikal oder

$R^1$ und $R^2$ zusammen mit dem Stickstoffatom, an das sie gebunden sind, einen 5-, 6- oder 7gliedrigen gesättigten heteromonocyclischen Ring, der eine Hydroxygruppe an einem dem Stickstoffatom nicht direkt benachbarten C-Atom tragen kann und der ein weiteres Sauerstoff-, Schwefel- oder Stickstoffatom enthalten kann, das ggf. durch eine Niederalkyl-, Hydroxy-niederalkyl-, Aryl- oder Aryl-niederalkylgruppe substituiert ist, darstellen

und von deren Säureadditionssalzen, dadurch gekennzeichnet, daß man

(a) ein Epoxid der Formel

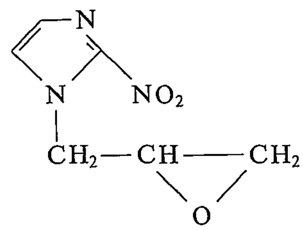

(II)

mit einem Amin der Formel

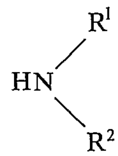

(III)

worin $R^1$ und $R^2$ wie oben definiert sind, umsetzt oder

(b) eine Verbindung der Formel

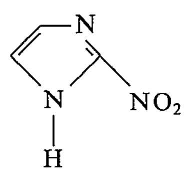

(IV)

mit einem Epoxid der Formel

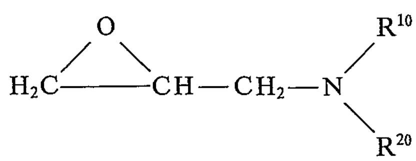

(V)

worin $R^{10}$ und $R^{20}$ ebenso definiert sind wie oben $R^1$ und $R^2$ mit dem Unterschied, daß $R^{10}$ nicht Wasserstoff ist, in Gegenwart einer Base umsetzt oder

(c) ein Halohydrin der Formel

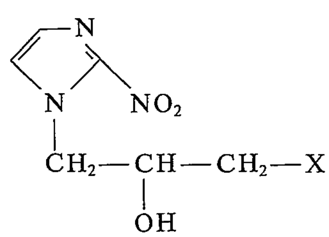

(VI)

worin X Chlor oder Brom darstellt, mit einem Amin der Formel III umsetzt und gewünschtenfalls eine erhaltene Verbindung I in ein Säureadditionssalz überführt.

2. Verfahren gemäß Anspruch 1, dadurch gekennzeichnet, daß man eine Verbindung der Formel I oder ein Säureadditionssalz davon herstellt, in der $R^1$ Wasserstoff, Niederalkyl, Hydroxy-niederalkyl, Aryl oder Arylniederalkyl ist; $R^2$ Niederalkyl, Hydroxy-niederalkyl, Aryl oder Aryl-niederalkyl ist oder $R^1$ und $R^2$ zusammen mit dem Stickstoffatom, an das sie gebunden sind, einen 5-, 6- oder 7gliedrigen gesättigten heteromonocyclischen Ring, der eine Hydroxygruppe an einem dem Stickstoffatom nicht direkt benachbarten C-Atom tragen kann und der ein weiteres Sauerstoff-, Schwefel- oder Stickstoffatom enthalten kann, das ggf. durch eine Niederalkyl-, Hydroxyniederalkyl-, Aryl- oder Aryl-niederalkyl-gruppe substituiert ist, darstellen.

3. Verfahren gemäß Anspruch 1, dadurch gekennzeichnet, daß man eine Verbindung der Formel I oder ein Säureadditionssalz davon herstellt, in der $R^1$ und $R^2$ gemeinsam mit dem Stickstoffatom, an das sie gebunden sind, einen 6gliedrigen heteromonocyclischen Ring, der ein weiteres Sauerstoff- oder Stickstoffatom enthalten kann, das ggf. durch eine Niederalkylgruppe substituiert ist, darstellen.

4. Verfahren zur Herstellung pharmazeutischer Präparate, dadurch gekennzeichnet, daß man eine in Anspruch 1 definierte Verbindung der Formel I, wie in Anspruch 1 definiert als aktiven Bestandteil mit einem zur therapeutischen Verabreichung geeigneten, inerten, festen oder flüssigen Träger vermischt und dem Gemisch ggf. eine besondere galenische Form gibt.

5. Pharmazeutische Präparate auf der Basis einer Verbindung der Formel I, wie in Anspruch 1 definiert.

6. Nitroimidazole der Formel I gemäß Anspruch 1 und deren Säureadditionssalze.

7. Verbindungen gemäß Anspruch 6, dadurch gekennzeichnet, daß in Formel I $R^1$ Wasserstoff, Niederalkyl, Hydroxy-niederalkyl, Aryl oder Aryl-niederalkyl ist; $R^2$ Niederalkyl, Hydroxy-niederalkyl, Aryl oder Arylniederalkyl ist oder $R^1$ und $R^2$ zusammen mit dem Stickstoffatom, an das sie gebunden sind, einen 5-, 6- oder 7gliedrigen gesättigten heteromonocyclischen Ring, der eine Hydroxygruppe an einem dem Stickstoffatom nicht direkt benachbarten C-Atom tragen kann und der ein weiteres Sauerstoff-, Schwefel- oder Stickstoffatom enthalten kann, das ggf. durch eine Niederalkyl-, Hydroxy-niederalkyl-, Aryl- oder Aryl-niederalkyl-gruppe substituiert ist, darstellen.

8. Verbindungen gemäß Anspruch 6, dadurch gekennzeichnet, daß in Formel I $R^1$ und $R^2$ gemeinsam mit dem Stickstoffatom, an das sie gebunden sind, einen 6gliedrigen heteromonocyclischen Ring, der ein weiteres Sauerstoff- oder Stickstoffatom enthalten kann, das ggf. durch eine Niederalkylgruppe substituiert ist, darstellen.

9. 2-Nitro-α-(piperidino)-methyl-1-imidazol-äthanol.

10. α-(Morpholino)-methyl-2-nitro-1-imidazol-äthanol.

11. α-(4-Methylpiperazino)-methyl-2-nitro-1-imidazol-äthanol.

12. 2-Nitro-α-(pyrrolidino)-methyl-1-imidazol-äthanol.

13. α-(Diäthylamino)-methyl-2-nitro-1-imidazol-äthanol.

14. α-[Di-(2-hydroxyäthyl)-amino]-methyl-2-nitro-1-imidazol-äthanol.

15. α-(tert.-Butylamino)-methyl-2-nitro-1-imidazol-äthanol.

16. α-(Benzylamino)-methyl-2-nitro-1-imidazol-äthanol.

17. α-[(4-Methoxyphenyl)-amino]-methyl-2-nitro-1-imidazol-äthanol.

18. α-(Dimethylamino)-methyl-2-nitro-1-imidazol-äthanol.

19. α-(Hexahydro-1 H-azepino)-methyl-2-nitro-1-imidazol-äthanol.

20. 4-[2-Hydroxy-3-(2-nitro-1-imidazolyl)-propyl-amino]-2,2,6,6-tetramethylpiperidin-N-oxyl.

21. α-[(2,2,6,6-Tetramethyl-4-piperidinyl)-amino]-methyl-2-nitro-1-imidazol-äthanol.

22. α-(Cyclohexylamino)-methyl-2-nitro-1-imidazol-äthanol.

23. α-(Dicyclohexylamino)-methyl-2-nitro-1-imidazol-äthanol.

24. 1-[2-Hydroxy-3-(2-nitro-1-imidazolyl)-propyl]-3-pyrrolidinol.

**Claims**

1. A process for the manufacture of nitroimidazoles of the formula

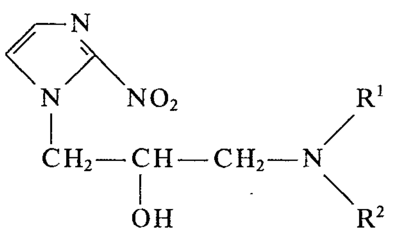

(I)

wherein $R^1$ represents hydrogen, lower alkyl, hydroxy- (lower alkyl), lower cycloalkyl, aryl or aryl-(lower alkyl), $R^2$ represents lower alkyl, hydroxy-(lower alkyl), lower cycloalkyl, aryl or aryl-(lower

alkyl) or a grouping of the formula

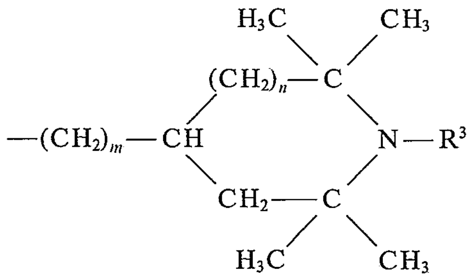

in which m=0 and n=1 or m=1 and n=0, $R^3$ represents hydrogen, methyl, hydroxy or a free oxygen radical, or $R^1$ and $R^2$ together with the nitrogen atom to which they are attached represent a 5-, 6- or 7membered saturated heteromonocyclic ring, which may carry a hydroxy group on a carbon atom not adjacent to the nitrogen atom and which may contain a further oxygen, sulphur or nitrogen atom, which may be substituted by a lower alkyl, hydroxy-(lower alkyl), aryl or aryl-(lower alkyl) group, and of acid addition salts thereof, characterized in that

(a) an epoxide of the formula

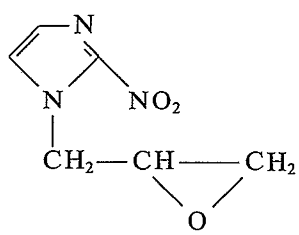

(II)

is reacted with an amine of the formula

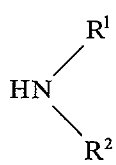

(III)

wherein $R^1$ and $R^2$ have the significance given above,

(b) a compound of the formula

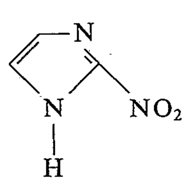

(IV)

is reacted with an epoxide of the formula

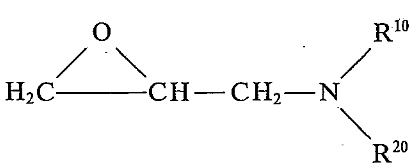

(V)

wherein $R^{10}$ and $R^{20}$ have any of the values accorded to $R^1$ and $R^2$ above except that $R^{10}$ does not represent hydrogen,
in the presence of a base, or

(c) a halohydrin of the formula

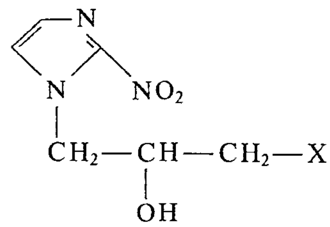

(VI)

wherein X represents chlorine or bromine,
is reacted with an amine of formula III and, if desired, a compound of formula I obtained is converted into an acid addition salt.

2. A process according to claim 1, characterized in that there is manufactured a compound of formula I, or an acid addition salt thereof, in which $R^1$ represents hydrogen, lower alkyl, hydroxy-(lower alkyl), aryl or aryl-(lower alkyl); $R^2$ represents lower alkyl, hydroxy-(lower alkyl), aryl or aryl-(lower alkyl) or $R^1$ and $R^2$ together with the nitrogen atom to which they are attached represent a 5-, 6- or 7membered saturated heteromonocyclic ring, which may carry a hydroxy group on a carbon atom not adjacent to the nitrogen atom and which may contain a further oxygen, sulphur or nitrogen atom, which may be substituted by a lower alkyl, hydroxy-(lower alkyl), aryl or aryl-(lower alkyl) group.

3. A process according to claim 1, characterized in that there is manufactured a compound of formula I or an acid addition salt thereof, in which $R^1$ and $R^2$ together with the nitrogen atom to which they are attached represent a 6membered heteromonocyclic ring, which may contain a further oxygen or nitrogen atom, which may be substituted by a lower alkyl group.

4. A process for the manufacture of pharmaceutical preparations, characterized in that a compound of the formula I as defined in claim 1, as the active ingredient, is mixed which an inert, therapeutically compatible solid or liquid carrier and that the mixture is given a suitable galenic form.

5. Pharmaceutical preparations containing a compound of the formula I as set forth in claim 1.

6. Nitroimidazoles of formula I given in claim 1 and their acid addition salts.

7. Compounds according to claim 6, characterized in that in formula I $R^1$ represents hydrogen, lower alkyl, hydroxy-(lower alkyl), aryl or aryl-(lower alkyl); $R^2$ represents lower alkyl, hydroxy-(lower alkyl), aryl or aryl-(lower alkyl) or $R^1$ and $R^2$ together with the nitrogen atom to which they are attached represent a 5-, 6- or 7membered saturated heteromonocyclic ring, which may carry a hydroxy group on a carbon atom not adjacent to the nitrogen atom and which may contain a further oxygen, sulphur or nitrogen atom, which may be substituted by a lower alkyl, hydroxy-(lower alkyl), aryl or aryl-(lower alkyl) group.

8. Compounds of formula I given in claim 6, characterized in that in formula I $R^1$ and $R^2$ together with the nitrogen atom to which they are attached represent a 6membered heterocyclic ring which may contain a further oxygen or nitrogen atom, which may be substituted by a lower alkyl group.

9. 2-Nitro-α-(piperidino)methyl-1-imidazole-ethanol.

10. α-(Morpholino)methyl-2-nitro-1-imidazole-ethanol.

11. α-(4-Methylpiperazino)methyl-2-nitro-1-imidazole-ethanol.

12. 2-Nitro-α-(pyrrolidino)methyl-1-imidazole-ethanol.

13. α-(Diethylamino)methyl-2-nitro-1-imidazole-ethanol.

14. α-[Di-(2-hydroxyethyl)amino]methyl-2-nitro-1-imidazole-ethanol.

15. α-(Tert.-butylamino)methyl-2-nitro-1-imidazole-ethanol.

16. α-(Benzylamino)methyl-2-nitro-1-imidazole-ethanol.

17. α-[(4-Methoxyphenyl)amino]methyl-2-nitro-1-imidazole-ethanol.

18. α-(Dimethylamino)methyl-2-nitro-1-imidazole-ethanol.

19. α-(Hexahydro-1 H-azepino)methyl-2-nitro-1-imidazole-ethanol.

20. 4-[2-Hydroxy-3-(2-nitro-1-imidazolyl)propylamino]-2,2,6,6-tetramethylpiperidin-N-oxyl.

21. α-[(2,2,6,6-Tetramethyl-4-piperidinyl)amino]methyl-2-nitro-1-imidazole-ethanol.

22. α-(Cyclohexylamino)methyl-2-nitro-1-imidazole-ethanol.

23. α-(Dicyclohexylamino)methyl-2-nitro-1-imidazole-ethanol.

24. 1-[2-Hydroxy-3-(2-nitro-1-imidazolyl)propyl]-3-pyrrolidinol.

**Revendications**

1. Un procédé pour la préparation de nitroimidazoles de formule générale

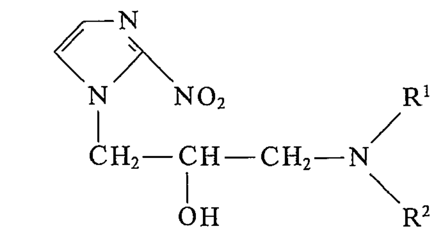

(I)

dans laquelle $R^1$ représente un atome d'hydrogène ou un groupe alcoyle inférieur, hydroxy-(alcoyle inférieur), cycloalcoyle inférieur, aryle ou aryl-(alcoyle inférieur) et $R^2$ représente un groupe alcoyle inférieur, hydroxy-(alcoyle inferieur), cycloalcoyle inférieur, aryle ou aryl-(alcoyle inférieur) ou un groupement de la formule

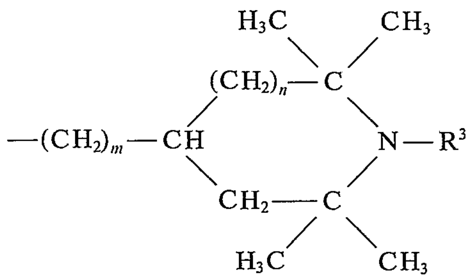

dans laquelle m représente zéro et n représente I ou m représente I et n représente zéro et $R^3$ représente un atome d'hydrogène ou le groupe méthyle ou hydroxy ou un radical libre d'oxygène, ou $R^1$ et $R^2$ représentent avec l'atome d'azote sur lequel ils sont fixés un noyau hétéromonocyclique saturé pentagonal, hexagonal ou heptagonal qui peut porter un groupe hydroxy sur un atome de carbone qui n'est pas lié directement à l'atome d'azote et qui peut contenir un autre atome d'oxygène, de soufre ou d'azote pouvant être substitué par un groupe alcoyle inférieur, hydroxy-(alcoyle inférieur), aryle ou aryl-(alcoyle inférieur),
et de leurs sels d'addition d'acide, caractérisé par le fait que

a) on fait réagir l'époxyde de formule

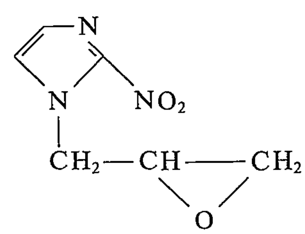

(II)

avec une amine de formule

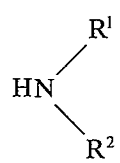

(III)

dans laquelle $R^1$ et $R^2$ ont la signification indiquée ci-dessus, ou

b) on condense le composé de la formule

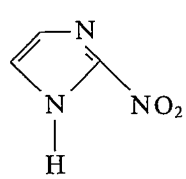

(IV)

avec un époxyde de formule

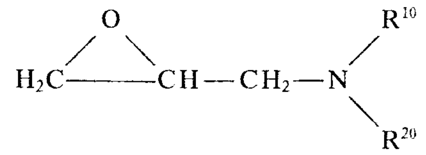

(V)

dans laquelle $R^{10}$ et $R^{20}$ sont définis comme $R^1$ et $R^2$ cidessus, à ceci près que $R^{10}$ ne représente pas un atome d'hydrogène, en présence d'une base, ou

c) on fait réagir une halohydrine de formule

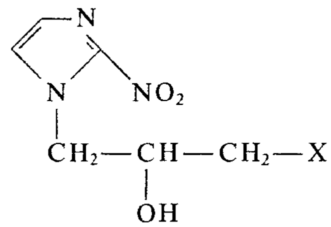

(VI)

dans laquelle X représente un atome de chlore ou de brome, avec une amine de formule III et, le cas échéant, on transforme un composé de formule I obtenu en un sel d'addition d'acide.

2. Un procédé selon la revendication 1, caractérisé en ce qu'on prépare un composé de formule I ou l'un de ses sels d'addition d'acide, où $R^1$ représente un atome d'hydrogène ou un groupe alcoyle inférieur, hydroxy-(alcoyle inférieur), aryle ou aryl-(alcoyle inférieur); $R^2$ représente un groupe alcoyle inférieur, hydroxy-(alcoyle inférieur), aryle ou aryl-(alcoyle inférieur) ou $R^1$ et $R^2$ représentent avec l'atome d'azote sur lequel ils sont fixés un noyau hétéromonocyclique saturé pentagonal, hexagonal ou heptagonal qui peut porter un groupe hydroxy sur un atome de carbone qui n'est pas lié directement à l'atome d'azote et qui peut contenir un autre atome d'oxygène, de soufre ou d'azote pouvant être substitué par un groupe alcoyle inférieur, hydroxy-(alcoyle inférieur), aryle ou aryl-(alcoyle inférieur).

3. Un procédé selon la revendication 1, caractérisé en ce qu'on prépare un composé de formule I ou l'un de ses sels d'addition d'acide, où $R^1$ et $R^2$ forment avec l'atome d'azote sur lequel ils sont fixés un noyau hétéromonocyclique hexagonal, qui peut contenir un autre atome d'oxygène ou d'azote, qui peut être substitué par un groupe alcoyle inférieur.

4. Un procédé pour la préparation d'une composition pharmaceutique, caractérisé en ce qu'on mélange un composé de formule I tel que défini dans la revendication 1, comme défini à la revendication 1, comme ingrédient actif, avec un véhicule inerte, solide ou liquide et donne au mélange, le cas échéant, une forme galénique spéciale.

5. Compositions pharmaceutiques sur la base d'un composé de formule I, comme défini à la revendication 1.

6. Nitroimidazoles de formule I selon la revendication 1 et leurs sels d'addition d'acide.

7. Composés selon la revendication 6, caractérisés en ce que dans la formule I $R^1$ représente un atome d'hydrogène ou un groupe alcoyle inférieur, hydroxy-(alcoyle inférieur), aryle ou aryl-(alcoyle inférieur); $R^2$ représente un groupe alcoyle inférieur, hydroxy-(alcoyle inférieur), aryle ou aryl-(alcoyle inférieur) ou $R^1$ et $R^2$ représentent avec l'atome d'azote sur lequel ils sont fixés un noyau hétéromonocyclique saturé pentagonal, hexagonal ou heptagonal qui peut porter un groupe hydroxy sur un atome de carbone qui n'est pas lié directement à l'atome d'azote et qui peut contenir un autre atome d'oxygène, de soufre ou d'azote pouvant être substitué par ún groupe alcoyle inférieur, hydroxy-(alcoyle inférieur), aryle ou aryl-(alcoyle inférieur).

8. Composés selon la revendication 6, caractérisé en ce que dans la formule I $R^1$ et $R^2$ représentent avec l'atome d'azote sur lequel ils sont fixés un noyau hétéromonocyclique saturé hexagonal, qui peut contenir un autre atome d'oxygène ou d'azote, qui peut être substitué par un groupe alcoyle inférieur.

9. Le 2-nitro-alpha-(pipéridino)méthyl-1-imidazoleéthanol.

10. L'alpha-(morpholino)méthyl-2-nitro-1-imidazole-éthanol.

11. L'alpha-(4-méthylpipérazino)méthyl-2-nitro-1-imidazole-éthanol.

12. Le 2-nitro-alpha-(pyrrolidino)méthyl-1-imidazole-éthanol.

13. L'alpha-(diéthylamino)méthyl-2-nitro-1-imidazole-éthanol.

14. L'alpha-(di-(2-hydroxyéthyl)amino)méthyl-2-nitro-1-imidazole-éthanol.

15. L'alpha-(tert-butylamino)méthyl-2-nitro-1-imidazole-éthanol.

16. L'alpha-(benzylamino)méthyl-2-nitro-1-imidazole-éthanol.

17. L'alpha-( (4-méthoxyphényl)amino)méthyl-2-nitro-1-imidazole-éthanol.

18. L'alpha-(diméthylamino)méthyl-2-nitro-1-imidazole-éthanol.

19. L'alpha-(hexahydro-1 H-azépino)méthyl-2-nitro-1-imidazole-éthanol.
20. Le 4-(2-hydroxy-3-(2-nitro-1-imidazolyl)-propyl-amino)-2,2,6,6-tétraméthylpipéridin-N-oxyde.
21. L'alpha-(2,2,6,6-tétraméthyl-4-pipéridinyl)-amino)méthyl-2-nitro-1-imidazole-éthanol.
22. L'alpha-(cyclohexylamino)méthyl-2-nitro-1-imidazole-éthanol.
23. L'alpha-(dicyclohexylamino)méthyl-2-nitro-1-imidazole-éthanol.
24. Le 1-(2-hydroxy-3-(2-nitro-1-imidazolyl)propyl)-3-pyrrolidinol.